# EUROPEAN PATENT SPECIFICATION

(11) **EP 4 494 628 B1**
(45) Date of publication and mention of the grant of the patent: **23.09.2026**
(21) Application number: 23186756.5
(22) Date of filing: 20.07.2023
(51) Int. Cl.: A61K 8/02, A61K 8/04, A61K 8/24, A61K 8/25, A61K 8/365, A61K 8/43, A61K 8/49, A61K 8/90, A61Q 11/00

(54) **KIT FOR PREPARING A COMPOSITION FOR THE DESENSIBILISATION OF TEETH**
KIT ZUR HERSTELLUNG EINER ZUSAMMENSETZUNG ZUR DESENSIBILISIERUNG VON ZÄHNEN
KIT DE PRÉPARATION D'UNE COMPOSITION POUR LA DÉSENSIBILISATION DES DENTS

(43) Date of publication of application: 22.01.2025
(73) Proprietor: Ferton Holding S.A., 2800 Delémont (CH)
(72) Inventor: Kratky, Julia, 64673 Zwingenberg (DE); Hisaschi, Tee, 64673 Zwingenberg (DE); Rizzi, Simone, 2800 Delémont (FR)
(74) Representative: Müller Schupfner & Partner Patent- und Rechtsanwaltspartnerschaft mbB (Muc)

(56) References cited:
- WO-A1-2021/001529
- DATABASE GNPD [online] MINTEL; 1 February 2021 (2021-02-01), ANONYMOUS: "Professional Toothpastes Set", XP093111209, retrieved from https://www.gnpd.com/sinatra/recordpage/8447347/ Database accession no. 8447347

## Description

The invention relates to a kit for preparing a composition for the desensibilisation of teeth comprising a dispersion of silica particles in water and calcium phosphate glass particles. The invention further relates to the use of the kit in the desensibilisation of teeth and a process for preparing a composition for the desensibilisation of teeth.

Dentin tubules are microscopic channels that extend from the tooth inside through the dentin and end below the enamel. Dental hypersensitivity may occur when dentin tubules are open as a result of a damaged enamel or gingival recession. Such dental hypersensitivity can be treated by a desensibilisation of the teeth, wherein the tubules are occluded or a sealing film is formed which inhibits stimuli resulting in a reduction or elimination of the hypersensitivity of the teeth.

WO 2019/034348 A1 discloses an oral care composition comprising a bioactive glass, a water soluble calcium source, a phosphate source and a physiologically acceptable carrier, wherein the bioactive glass and the water soluble calcium source are present in a weight ratio (a:b) from 1:3 to 20:1, and wherein the calcium source is calcium chloride, calcium nitrate, calcium gluconate, calcium glycerophosphate or mixtures thereof.

A product for desensibilisation of the teeth which works as a teeth desensitizer is described in WO 2021/001529 A1. This product contains two main ingredients, a dispersion of silica particles and calcium phosphate glass. The product is a paste that can be spread on the teeth and exposed dentine. The silica dispersion forms a dried gel layer with fine calcium phosphate glass particles that protects the dentine on the surface and occludes the dentine tubules.

The preparation time of this product, however, is about 14 hours. This time is required to reach a product paste consistency that can be spread during the final application onto the teeth. The first step involves mixing of the colloidal silica dispersion with water (1:2) for 15 minutes (liquid component). To this liquid component CaP glass is added and stirred for 1 h at 700-750 rpm. Furthermore, diluted HCl is added and the suspension is stirred overnight. Finally, chlorhexidine (CHX) digluconate solution (20%) is added and stirred for 5 minutes. Precipitation occurs and after at least 2 h the mixture solidifies (gelation time).

Even though this prototype described in WO 2021/001529 A1 was successful in occluding the dentine tubules and decreasing hypersensitivity in patients, a long-term storage was not possible as the product deteriorates after about 30 days. This makes the product not well handleable by end users. If a product is prepared by the end user, handling should be easy and the preparation time and gelation time should be short. The gelation time of the prototype was two hours and preparation time 14 hours which is too long for a useful product to be handled.

In view of the above, it is an object of the present invention to provide a composition for the desensibilisation of teeth comprising silica particles and calcium phosphate that can be long-term stored and has a short preparation time and gelation time.

This object is achieved by a kit for preparing a composition for the desensibilisation of teeth comprising
A) a first component comprising a colloidal dispersion of silica particles in water,
B) a second component being a composition, optionally presented in two or more parts, comprising calcium phosphate glass particles, a surfactant and an acid.

The object is also achieved by the above kit for use in the desensibilisation of teeth. The object is further achieved by a use of the kit for preparing a composition for the desensibilisation of teeth and the object is achieved by a process for preparing a composition for the desensibilisation of teeth comprising the steps that the first component and the second component of the kit according to the invention are mixed.

Preferred embodiments of the invention are subject to the dependent claims as well as the following description.

The kit according to the invention comprises A) a first component and B) a second component. The first component is a liquid dispersion and the second component may be a powder, granular or in the form of a concentrate or slurry. The second component is preferably a powder or granular. The kit according to the invention is understood that the first component and the second component are separated, for example by chambers of a container or via tubs, bags and sachets in a box. The kit is preferably in the form of a multi-chambered container. This allows that the components are easily mixed and the kit is very usable for the end user.

The kit according to the invention is for use in the desensibilisation of teeth. For this use, the components are mixed and the resulting mixture is applied to teeth. The application can be accomplished for example with a brush or a cotton swab. A film or gel layer is formed which contains the calcium phosphate glass particles. The gel layer with the calcium phosphate glass particles hardens on the teeth, preferably by drying with additional air flow, and seals the surface of the exposed dentin tubules. This procedure seals the dentine tubules and reduces or stops the dental hypersensitivity.

It has surprisingly been found that the separation of the silica particle dispersion on the one hand and the calcium phosphate glass on the other hand, by presenting a first and a second component in the kit, gives a stable kit that can be stored for a long term. Further, it has surprisingly been found that the addition of a surfactant and an acid, preferably an organic acid leads to a composition that after mixing of all components of the kit has a short gelation time of less than two minutes. Further, the preparation time for the user is very short. This makes the composition very useful for the desensibilisation of teeth, for example at the dentist or an end user. In total, the kit according to the invention is very viable from different points of view, for example the preparation time, storage and the user handling. Further, the obtained mixture (gel) after mixing the components of the kit can be stored at 2 - 8 °C for up to one month and can be re-used for another application on the teeth.

It has been found that the separation of the first component from the other components avoids unwanted and premature reaction of the silica particle dispersion, for example during storage. This reaction is called gelation. It has further been found that the organic acid allows a very controlled reduction of the pH, in particular below 7. The pH is preferably 2 to 7.2. More preferably, the pH is 6 - 6.9, even more preferably 6.2 - 6.6. These moderate pH values furthermore allow a particularly safe application for end users on the teeth. The acidic pH starts the gelation reaction and it has been found that a further ingredient is required which induces the destabilization of the silica particles so that the gelation is triggered. According to the invention, this additional ingredient is a surfactant.

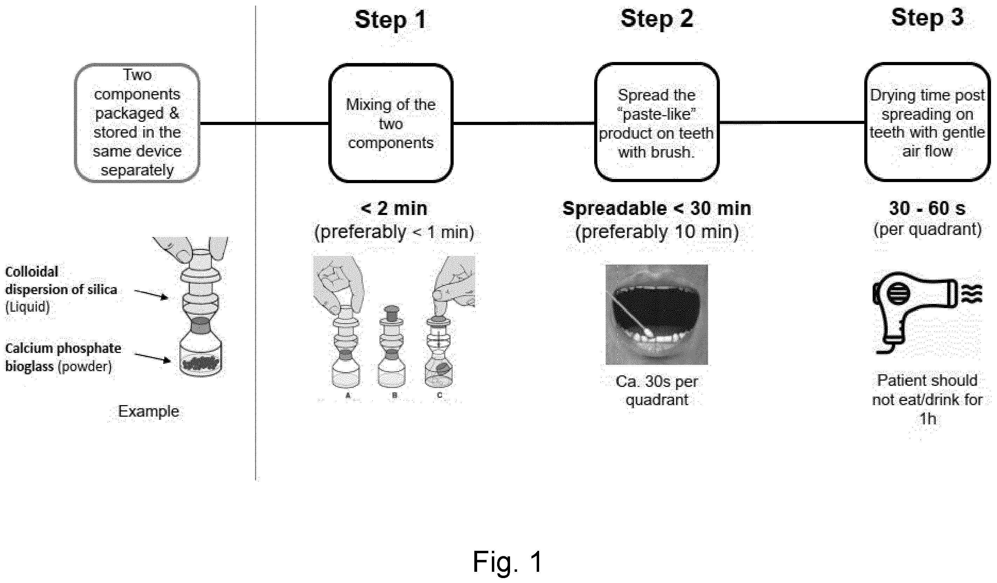

Fig. 1 shows a schematic overview of the use of the composition according to the invention. The first component and the second component are stored in the same device in separated chambers. In a first step, the two components are brought together and mixed for less than 2 min. Preferably, the two components are brought together and mixed for less than a minute. In a second step, the resulting mixture is allowed to stand for up to 30 min, preferably 10 min, and the resulting mixture in the form of a paste is spread on the teeth with a brush. In the next step, the paste on the teeth is dried with gentle air flow for about 30 - 60 s.

The colloidal dispersion of silica particles in water can also be named a silica sol. Such colloidal dispersion of silica particles in water is commercially available, for example under the name KÖSTROSOL^{®}, manufactured by the company Bad Köstritz GmbH. This product KÖSTROSOL^{®} usually comprises 15 - 50 wt.-% amorphous silica and 44.5 - 84.5 wt.-% water and can be further diluted with water.

In the present invention, the dispersion of silica particles in water comprises preferably 3 - 25 wt.-%, more preferably 5 - 20 wt.-%, silica particles and 75 - 97 wt.-%, preferably 80 - 95 wt.-% water. A colloid or colloidal dispersion according to the invention is understood as particles that are finely dispersed in the dispersion medium. The size of the particles is preferably in the range of one nanometer to one micrometer. i.e. 1 - 1000 nm. The particles size of the silica particles is preferably determined by sieve analysis, as described below, wherein the particle size distribution is based on the mass.

A preferred preparation method of the calcium phosphate glass particles is described in WO 2021/001529 A1 and the content of this patent application is incorporated herein by reference. To shortly summarize this preparation method of calcium phosphate glass: CaCO₃ and P₂O₂ are mixed, for example in a mortar, the resulting powder is melted in a furnace, for example at around 1000 °C, the molten glass is then poured into water and the glass is then preferably ground and optionally sieved to have glass particles with a certain maximum particle size, for example preferably < 10µm.

The calcium phosphate glass particles are preferably a powder. The calcium phosphate glass particles preferably have a maximum particle size of less than 130 µm (d₁₀₀ < 130 µm), more preferably d₁₀₀ < 120 µm, most preferably d₁₀₀ < 110 µm. The particles size is preferably determined by sieve analysis, for example with a sieve tower, wherein the particle size distribution is based on the mass (weight). This means, for example, when d₁₀₀ is less than 130 µm that 100 wt.-% (weight percent) of the particles have a size less than 130 µm. The sieve analysis is described, for example, in the German industry norm DIN 66165.

The d₉₀ of the calcium phosphate glass particles is preferably < 90 µm, more preferably < 20 µm. Further, d₅₀ is preferably < 35 µm, more preferably < 20 µm. d₉₀ and d₅₀ can also be determined by the above sieve analysis. Alternatively, it is possible to determine these values by laser granulometry, which also gives particle size distributions based on the weight. Sieve analysis is preferred.

In a preferred embodiment of the invention, the surfactant is selected from the group consisting of pluronic F127, lecithin, cetylpyridinium chloride (CPC), chlorhexidine (CHX) diacetate, chlorhexidine (CHX) digluconate, olaflur, sodium coco sulfate (SCS) or mixtures thereof. The surfactant is selected from the group consisting of pluronic F127, cetylpyridinium chloride (CPC), chlorhexidine diacetate, or mixtures thereof. These surfactants have found to give together with an acid, preferably an organic acid a very preferred short gelation time of about 1 - 2 minutes.

An organic acid according to the invention is an acid that has a carbon skeleton, for example acetic acid or lactic acid. Preferred organic acids are organic carboxylic acids and organic sulfonic acids, more preferred are organic carboxylic acids.

The acid can be an inorganic acid or an organic acid. An organic acid is preferred. The acid, preferably the organic acid, is preferably a powder. This allows additionally to combine the acid or organic acid with the other ingredients of the second component, i.e. the calcium phosphate glass particles the surfactant without reducing the stability of the kit.

In a preferred embodiment of the invention, the second component is presented in one part and each of calcium phosphate glass particles, surfactant and acid are in powder form or the second component is presented in two parts, wherein one part comprises the calcium phosphate glass particles and the surfactant and a second part comprises the acid.

In a preferred embodiment of the invention, the calcium phosphate glass particles, the surfactant and/or the acid, preferably the organic acid, are powders.

The organic acid is preferably selected from the group consisting of lactic acid, gluconic acid, tartaric acid, citric acid, alginic acid, glycolic acid, aspartic acid, ascorbic acid, sorbic acid, or mixtures thereof. More preferably, the organic acid is selected from the group consisting of lactic acid and gluconic acid.

In a preferred embodiment of the invention, the kit comprises 89.5 - 97.97 wt.-% of the first component and 2.03 - 10.5 wt.-% of the second component.

Further, it is preferred that the kit according to the invention comprises
89.5 - 97.97 wt.-% of the first component and
2 - 8 wt.-%, preferably 3 - 7 wt.-%, more preferably 4 - 5.5 wt.-% calcium phosphate particles,
0.01 - 2 wt.-%, preferably 0.05 - 1.5 wt.-%, more preferably 0.1 - 1 wt.-% surfactant and
0.02 - 2 wt.-%, preferably 0.05 - 1 wt.-%, more preferably 0.1 - 0.5 wt.-% acid, preferably organic acid.

The invention further relates to the kit as described herein for use in the desensibilisation of teeth. Accordingly, the invention also relates to a kit for use in the desensibilisation of teeth comprising
A) a first component comprising a colloidal dispersion of silica particles in water,
B) a second component being a composition, optionally presented in two or more parts, comprising calcium phosphate glass particles, a surfactant and an acid, preferably an organic acid.

The invention also relates to the use of the kit according to the invention for preparing a composition for the desensibilisation of teeth.

The invention also relates to a process for preparing a composition for the desensibilisation of teeth comprising the steps that the first component and the second component of the kit according to the invention are mixed. The mixing can be a shaking or stirring. The mixing is preferably conducted for about 0.5 to 2 minutes, more preferably 10 - 45 seconds, even more preferably 15 - 30 seconds. Afterward, the resulting mixture is applied on teeth and the applied mixture on the teeth is dried, preferably for about 30 to 90 seconds. The drying can be supported by an air stream, for example dried with a blow dryer.

The following examples provide preferred embodiments according to the invention and further exemplify the invention.

### Examples

The following compositions were prepared:

### List of ingredients

| Components (concentration of used component in end formulation) | One component (not according to the invention) | Two component (according to the invention) | | | |
|---|---|---|---|---|---|
| | Formulation 1 | Formulation 2 | Formulation 3 | Formulation 4 | Formulation 5 |
| Köstrosol | 30.8 % | 31.3 % | 31,9 % | 18.8 % | 18.8 % |
| Water | 61.1 % | 62.5 % | 63.1 % | 75.4 % | 75.3 % |
| CHX-Digluconate (20%, solution) | 1 % | - | - | - | - |
| HCl(2.14%) | 4.8 % | - | - | - | - |
| Lactic acid | - | - | - | - | 0.14 % |
| Glycolic acid | - | 0.24 % | 0.27 % | 0.16 % | - |
| CaP glass | 2.3 % | 4.6 % | 4.6 % | 4.7 % | 4.7 % |
| Pluronic F127 | - | 0.5 | - | - | - |
| KCl | - | - | - | - | - |
| CPC | - | - | 0.1 % | - | - |
| CHX diacetate | - | - | - | 0,1 % | 0,1 % |
| Gelation time (Min) | ~2-12 h | <2 min | <2 min | <2 min | <2 min |

The production procedure of the above one-component kit (formulation 1) is too complex and too long for practical use as it takes about 14 h. Further, stability issues arise so that the composition cannot be stored for more than about 30 to 90 days without severe destabilization. In addition, the gelation time is too long for a practical handling.

Formulations 2 - 5 (two component kit) show a good long-term storability without degradation and a short gelation time compared to the one-component kit.

Formulations 2 - 5 show the same good ability to occlude the dentine tubules and the same ability to minimize unevenness and defects on the dentine surface as those in WO 2021/001529 A1.

## Claims

1. Kit for preparing a composition for the desensibilisation of teeth comprising
A) a first component comprising a colloidal dispersion of silica particles in water,
B) a second component being a composition, optionally presented in two or more parts, comprising calcium phosphate glass particles, a surfactant and an acid.

2. Kit according to claim 1, **characterized in that** the acid is an organic acid.

3. Kit according to claim 1 or 2, **characterized in that** the calcium phosphate glass particles have a maximum particle size < 130 µm, based on the weight of the particles and determined by sieve analysis, preferably less than 120 µm.

4. Kit according to any of the preceding claims, wherein the surfactant is selected from the group consisting of lecithin, cetylpyridinium chloride (CPC), chlorhexidine diacetate, chlorhexidine digluconate, olaflur, sodium coco sulfate (SCS) or mixtures thereof.

5. Kit according to any of the preceding claims, **characterized in that** the surfactant is selected from the group consisting of cetylpyridinium chloride (CPC), chlorhexidine diacetate, or mixtures thereof.

6. Kit according to any of the preceding claims, **characterized in that** the organic acid is a powder.

7. Kit according to any of the preceding claims, **characterized in that** the second component is presented in one part and each of calcium phosphate glass particles, surfactant and acid are in powder form or the second component is presented in two parts, wherein one part comprises the calcium phosphate glass particles and the surfactant and a second part comprises the acid.

8. Kit according to any of the preceding claims, **characterized in that** each of calcium phosphate glass particles, surfactant and acid are in powder form.

9. Kit according to any of the preceding claims, **characterized in that** the acid is selected from the group consisting of lactic acid, gluconic acid, tartaric acid, citric acid, alginic acid, glycolic acid, aspartic acid, ascorbic acid, sorbic acid, or mixtures thereof, preferably **characterized in that** the acid is selected from the group consisting of lactic acid and gluconic acid.

10. Kit according to any of the preceding claims, **characterized in that** the kit comprises
89.5 - 97.97 wt.-% of the first component and
2.03 - 10.5 wt.-% of the second component.

11. Kit according to any of the preceding claims, **characterized in that** the kit comprises
89.5 - 97.97 wt.-% of the first component and
2 - 8 wt.-%, preferably 3 - 7 wt.-%, more preferably 4 - 5.5 wt.-% calcium phosphate particles,
0.01 - 2.0 wt.-%, preferably 0.05 - 1.5 wt.-%, more preferably 0.1 - 1 wt.-% surfactant and
0.02 - 2 wt.-%, preferably 0.05 - 1 wt.-%, more preferably 0.1 - 0.5 wt.-% acid.

12. Kit according to any of the preceding claims, **characterized in that** the first component comprises 3 - 25 wt.-% silica particles and 75 - 97 wt.-% water.

13. Kit according to any of the preceding claims for use in the desensibilisation of teeth.

14. Use of a kit according to any of the preceding claims for preparing a composition for the desensibilisation of teeth.

15. Process of preparing a composition for the desensibilisation of teeth comprising the steps that the first component and the second component of the kit according to any of claims 1 - 13 are mixed.

## Patentansprüche

1. Set zur Herstellung einer Zusammensetzung zur Desensibilisierung von Zähnen, umfassend
A) eine erste Komponente, die eine kolloidale Dispersion von Siliciumdioxidpartikeln in Wasser umfasst,
B) eine zweite Komponente, bei der es sich um eine Zusammensetzung handelt, die insbesondere in zwei oder mehr Teilen vorliegt, Calciumphosphat-Glaspartikel, ein Tensid und eine Säure umfasst.

2. Set gemäß Anspruch 1, **dadurch gekennzeichnet, dass** es sich bei der Säure um eine organische Säure handelt.

3. Set gemäß Anspruch 1 oder 2, **dadurch gekennzeichnet, dass** die Calciumphosphat-Glaspartikel eine maximale Partikelgröße von < 130 µm aufweisen, bezogen auf das Gewicht der Partikel und definiert durch Siebanalyse, vorzugsweise weniger als 120 µm.

4. Set gemäß einem der vorhergehenden Ansprüche, wobei das Tensid aus der Gruppe ausgewählt ist, die aus Lecithin, Cetylpyridiniumchlorid (CPC), Chlorhexidindiacetat, Chlorhexidindigluconat, Olaflur, Natriumkokosulfat (SCS) oder Mischungen davon besteht.

5. Set gemäß einem der vorhergehenden Ansprüche, **dadurch gekennzeichnet, dass** das Tensid aus der Gruppe ausgewählt ist, die aus Cetylpyridiniumchlorid (CPC), Chlorhexidindiacetat oder Mischungen davon besteht.

6. Set gemäß einem der vorhergehenden Ansprüche, **dadurch gekennzeichnet, dass** die organische Säure in Form von Pulver vorliegt.

7. Set gemäß einem der vorhergehenden Ansprüche, **dadurch gekennzeichnet, dass** die zweite Komponente einteilig vorliegt und sowohl die Calciumphosphat-Glaspartikel als auch das Tensid und die Säure in Pulverform vorliegen, oder dass die zweite Komponente zweiteilig vorliegt, wobei ein Teil die Calciumphosphat-Glaspartikel und das Tensid und ein zweiter Teil die Säure umfasst.

8. Set gemäß einem der vorhergehenden Ansprüche, **dadurch gekennzeichnet, dass** sowohl die Calciumphosphat-Glaspartikel als auch das Tensid und die Säure in Pulverform vorliegen.

9. Set gemäß einem der vorhergehenden Ansprüche, **dadurch gekennzeichnet, dass** die Säure aus der Gruppe ausgewählt ist, die aus Milchsäure, Gluconsäure, Weinsäure, Zitronensäure, Alginsäure, Glykolsäure, Asparaginsäure, Ascorbinsäure, Sorbinsäure oder Mischungen davon besteht, vorzugsweise **dadurch gekennzeichnet, dass** die Säure aus der Gruppe ausgewählt ist, die aus Milchsäure und Gluconsäure besteht.

10. Set gemäß einem der vorhergehenden Ansprüche, **dadurch gekennzeichnet, dass** der Werkzeugsatz
89,5 - 97,97 Gew.-% der ersten Komponente und
2,03 - 10,5 Gew.-% der zweiten Komponente umfasst.

11. Set gemäß einem der vorhergehenden Ansprüche, **dadurch gekennzeichnet, dass** der Werkzeugsatz
89,5 - 97,97 Gew.-% der ersten Komponente und
2 - 8 Gew.-%, vorzugsweise 3 - 7 Gew.-%, besonders bevorzugt 4 - 5,5 Gew.-% Calciumphosphat-Partikel,
0,01-2,0 Gew.-%, vorzugsweise 0,05-1,5 Gew.-%, besonders bevorzugt 0,1-1 Gew.-% Tensid und
0,02-2 Gew.-%, vorzugsweise 0,05-1 Gew.-%, besonders bevorzugt 0,1-0,5 Gew.-% Säure.

12. Set gemäß einem der vorhergehenden Ansprüche, **dadurch gekennzeichnet, dass** die erste Komponente 3-25 Gew.-% Siliciumdioxidpartikel und 75-97 Gew.-% Wasser umfasst.

13. Set gemäß einem der vorhergehenden Ansprüche zur Verwendung bei der Desensibilisierung von Zähnen.

14. Verwendung eines Sets gemäß einem der vorhergehenden Ansprüche zur Herstellung einer Zusammensetzung zur Desensibilisierung von Zähnen.

15. Verfahren zur Herstellung einer Zusammensetzung zur Desensibilisierung von Zähnen, umfassend die Schritte, bei denen die erste Komponente und die zweite Komponente des Sets gemäß einem der Ansprüche 1 bis 13 gemischt werden.

## Revendications

1. Kit destiné à la préparation d'une composition pour la désensibilisation des dents, comprenant :
A) un premier composant comprenant une dispersion colloïdale de particules de silice dans l'eau,
B) un deuxième composant qui est une composition se présentant éventuellement en deux parties ou plus, comprenant des particules de verre de phosphate de calcium, un agent tensioactif et un acide.

2. Kit selon la revendication 1,
**caractérisé en ce que** l'acide est un acide organique.

3. Kit selon la revendication 1 ou 2,
**caractérisé en ce que** les particules de verre de phosphate de calcium ont une taille maximale < 130 µm, calculée sur la base du poids des particules et déterminée par analyse granulométrique, de préférence inférieure à 120 µm.

4. Kit selon l'une des revendications précédentes,
dans lequel l'agent tensioactif est choisi parmi le groupe constitué de la lécithine, du chlorure de cétylpyridinium (CPC), du diacétate de chlorhexidine, du digluconate de chlorhexidine, de l'olaflur, du sodium coco-sulfate (SCS) ou de leurs mélanges.

5. Kit selon l'une des revendications précédentes,
**caractérisé en ce que** l'agent tensioactif est choisi parmi le groupe constitué du chlorure de cétylpyridinium (CPC), du diacétate de chlorhexidine ou de leurs mélanges.

6. Kit selon l'une des revendications précédentes,
**caractérisé en ce que** l'acide organique se présente sous forme de poudre.

7. Kit selon l'une des revendications précédentes,
**caractérisé en ce que** le deuxième composant se présente en une seule partie et **en ce que** les particules de verre de phosphate de calcium, l'agent tensioactif et l'acide se présentent chacun sous forme de poudre, ou bien **en ce que** le deuxième composant se présente en deux parties, l'une comprenant les particules de verre de phosphate de calcium et l'agent tensioactif, et l'autre comprenant l'acide.

8. Kit selon l'une des revendications précédentes,
**caractérisé en ce que** les particules de verre de phosphate de calcium, l'agent tensioactif et l'acide se présentent chacun sous forme de poudre.

9. Kit selon l'une des revendications précédentes,
**caractérisé en ce que** l'acide est choisi parmi le groupe constitué de l'acide lactique, de l'acide gluconique, de l'acide tartrique, de l'acide citrique, de l'acide alginique, de l'acide glycolique, de l'acide aspartique, de l'acide ascorbique, de l'acide sorbique ou de leurs mélanges, de préférence **caractérisé en ce que** l'acide est choisi parmi le groupe constitué de l'acide lactique et de l'acide gluconique.

10. Kit selon l'une des revendications précédentes,
**caractérisé en ce que** le kit comprend 89,5 à 97,97 % en poids du premier composant et 2,03 à 10,5 % en poids du deuxième composant.

11. Kit selon l'une des revendications précédentes,
**caractérisé en ce que** le kit comprend 89,5 à 97,97 % en poids du premier composant et 2 à 8 % en poids, de préférence 3 à 7 % en poids, plus préférablement 4 à 5,5 % en poids de particules de phosphate de calcium,
0,01 à 2,0 % en poids, de préférence 0,05 à 1,5 % en poids, plus préférablement 0,1 à 1 % en poids d'agent tensioactif et
0,02 à 2 % en poids, de préférence 0,05 à 1 % en poids, plus préférablement 0,1 à 0,5 % en poids d'acide.

12. Kit selon l'une des revendications précédentes,
**caractérisé en ce que** le premier composant comprend 3 à 25 % en poids de particules de silice et 75 à 97 % en poids d'eau.

13. Kit selon l'une des revendications précédentes, destiné à être utilisé pour la désensibilisation des dents.

14. Utilisation d'un kit selon l'une des revendications précédentes pour la préparation d'une composition destinée à la désensibilisation des dents.

15. Procédé de préparation d'une composition destinée à la désensibilisation des dents, comprenant les étapes consistant à mélanger le premier comsant et le deuxième composant du kit selon l'une des revendications 1 à 13.
